# EUROPEAN PATENT APPLICATION

(11) **EP 3 461 464 A1**
(43) Date of publication of application: **03.04.2019**
(21) Application number: 18193970.3
(22) Date of filing: 12.09.2018
(51) Int. Cl.: A61F 5/445

(54) **INTERNAL COLOSTOMY CATHETER**

(30) Priority: 13.09.2017 US 201762557930 P; 23.07.2018 US 201816042381
(71) Applicant: Covidien LP, Mansfield, MA 02048 (US)
(72) Inventor: AVVLN, Srinivasa Murthy Aravalli, 534211 Andhra Pradesh (IN)
(74) Representative: Maschio, Antonio

(57) **Abstract**

An internal colostomy catheter includes a pouch or bag that is secured to a body vessel such as the colon or intestine and remains within the abdomen to store fecal matter or urine. The internal colostomy catheter obviates the need for creation of a stoma and a colostomy bag secured to the stoma on an outer wall of the abdomen. The presently disclosed internal colostomy catheter includes exemplary embodiments of a sensor that monitor the fill rate and or level of fluid within the internal colostomy catheter and notify a patient when the internal colostomy catheter requires evacuation.

## Description

### CROSS-REFERENCE TO RELATED APPLICATIONS

This application claims the benefit of and priority to U.S. Provisional Patent Application No. 62/557,930 filed September 13, 2017, the entire disclosure of which is incorporated by reference herein.

### BACKGROUND

### 1. Technical Description

The present disclosure is directed to an internal colostomy catheter and, more particularly, to internal colostomy catheters that have fluid level sensors for identifying when the internal colostomy catheter requires evacuation.

### 2. Background of Related Art

During an ostomy procedure, a portion of an internal body vessel, such as the intestine or colon, is exteriorized to form a stoma. Stomas may be created in conjunction with an ostomy procedure by securing a bisected portion of the internal body vessel to the abdominal wall to provide internal access into the internal body vessel for collecting fecal matter.

Ostomy surgery is sometimes performed on an emergency basis due to diverticulitis, trauma, radiation complications, volvulus, necrotic bowel, bowel perforation, etc. Children and adults alike may require an ostomy. An ostomy may only be temporary to allow for healing of the bowel or a decrease of inflammation at the surgical site. In some instances an ostomy may be permanent.

In ostomy procedures, the internal body vessel is secured to the abdominal wall and/or cutaneous tissue of the abdomen. Securing the internal body vessel to the abdominal wall and/or cutaneous tissue of the abdomen keeps the stoma in the desired location and prevents it from withdrawing back into the abdominal cavity. Typically, a colostomy bag is connected to the stoma to collect waste materials from the internal body vessel.

Although a stoma has no sensory nerve endings and is insensitive to pain, several complications can result from the existence of a stoma, e.g., infection. As such, the condition of the stoma must be assessed regularly. In addition, the use of a colostomy bag secured to an outer surface of a patient's abdomen can reduce the quality of life of the patient.

Thus, there is a continuing need in the medical arts for an alternative mechanism for collecting fecal matter or waste material from an internal body vessel that overcomes the above disadvantages and can improve the quality of life of patient's requiring ostomy.

### SUMMARY

One aspect of the present disclosure is directed to a surgical method that includes securing a catheter to an internal body vessel within an abdominal cavity of a patient, the catheter having a body including an open end and a closed end and defining a reservoir, the open end being secured to the internal body vessel such that waste material from the internal body vessel is received within the reservoir; enclosing the catheter within the abdominal cavity; sensing the level of waste material within the reservoir with a sensor; and transmitting a signal from the sensor to an indication device positional externally of the abdominal cavity.

In certain embodiments, the method includes evacuating waste material from the reservoir through a drain tube supported on the catheter when the level of waste material exceeds a predetermined amount.

Another aspect of the present disclosure is directed to an internal colostomy catheter including a body defining a reservoir and having an open end and a closed end. The open end of the body is configured and dimensioned to be secured to an internal body vessel. A hollow drain tube defining a discharge channel communicates with the reservoir. A control valve is positioned within the discharge channel. A sensor is supported on the body to monitor a fluid level within the reservoir. A control unit processes a signal received from the sensor and transmits the signal to an indication device. The indication device is adapted to receive the signal transmitted from the control unit and provide an indication related to the fluid level within the reservoir.

In embodiments, the sensor includes a strain gauge that is positioned on the body of the catheter.

In some embodiments, the body of the catheter is formed of an expandable material and the strain gauge measures expansion of the expandable material.

In certain embodiments, the sensor includes a fluid sensor supported on the body of the catheter.

In embodiments, the fluid sensor is supported on a transparent indicator tube that extends from the body of the catheter, and the method further includes analyzing the indicator tube to check for the presence of waste material.

In some embodiments, the sensor includes an optical fluid level sensor supported on the body of the catheter adjacent the open end of the body of the catheter.

In certain embodiments, the sensor includes a pressure switch that is positioned on the closed end of the body of the catheter.

In some embodiments, transmitting the signal from the sensor on the catheter to the indication device is done wirelessly.

In certain embodiments, the body includes a flexible separation wall positioned atop the pressure sensor within the reservoir.

In embodiments, the indication device is selected from a group consisting of a watch and a cell phone.

In some embodiments, the indication device produces an audible indication.

In certain embodiments, the indication device produces a visual indication.

### BRIEF DESCRIPTION OF THE DRAWINGS

Various embodiments of the presently disclosed internal colostomy catheter are described herein below with reference to the drawings, wherein:
FIG. 1 is a side perspective view of an exemplary embodiment of the presently disclosed internal colostomy catheter including a first type of fluid level sensor;
FIG. 2 is a side perspective view of the internal colostomy catheter shown in FIG. 1 secured to an internal body vessel;
FIG. 3 is a side view of another exemplary embodiment of the presently disclosed internal colostomy catheter including a second type of fluid level sensor;
FIG. 4 is a side view of another exemplary embodiment of the presently disclosed internal colostomy catheter including a third type of fluid level sensor;
FIG. 5 is a side view of another exemplary embodiment of the presently disclosed internal colostomy catheter including a fourth type of fluid level sensor; and
FIG. 6 is a side view of another exemplary embodiment of the presently disclosed internal colostomy catheter including a fifth type of fluid level sensor.

### DETAILED DESCRIPTION OF EMBODIMENTS

The presently disclosed internal colostomy catheter will now be described in detail with reference to the drawings in which like reference numerals designate identical or corresponding elements in each of the several views. However, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

The presently disclosed internal colostomy catheter includes a body defining a reservoir that is secured to an internal body vessel such as the colon or intestine and remains within the abdomen to store waste material, i.e., fecal matter or urine. The internal colostomy catheter obviates the need for a colostomy bag that is secured to an outer wall of the abdomen. The presently disclosed internal colostomy catheter includes exemplary embodiments of a sensor that monitor the fill rate and or level of fluid within the internal colostomy catheter and notify a patient when the internal colostomy catheter is filled and requires evacuation.

Although the present disclosure is shown and described for use as a colostomy catheter, it is also envisioned that the internal catheter described herein may also be used within the urinary tract of a patient. For example, the presently disclosed catheter can be used to collect urine in patient's in which the bladder has been removed.

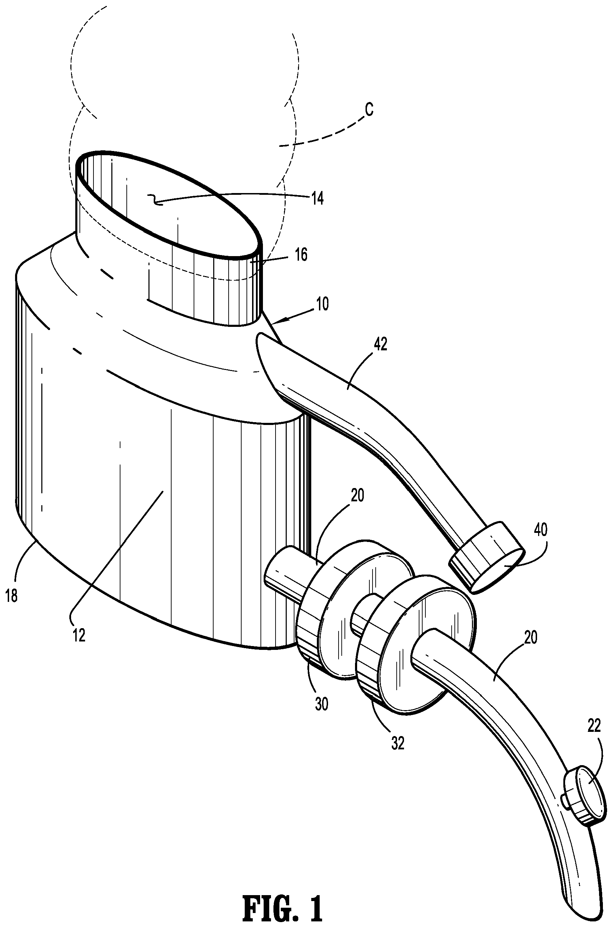

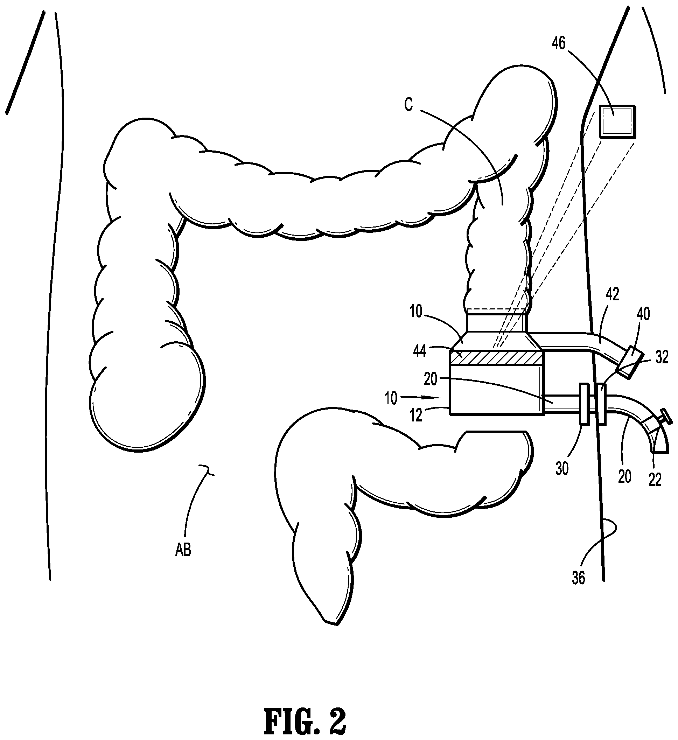

Referring to FIGS. 1 and 2, the internal colostomy catheter, shown generally as catheter 10, includes a body 12 defining a reservoir 14 and having an open end 16 and a closed end 18. The body 12 includes a drain tube 20 defining a discharge channel (not shown) that communicates with the reservoir 14 and is positioned near the closed end of the body 12. The drain tube 20 supports a control valve 22 that can be selectively opened or closed to open or close the discharge channel. The size of the reservoir 14 defined by the body 12 can be as big as a patient's natural rectal and colon reservoir. Alternately, the size of the reservoir 14 defined by the body 12 of the catheter 10 can be selected to suit a particular patient.

In embodiments, the body 12 is formed from an expandable and resilient biocompatible material such as an elastomeric polymer, capable of expanding as waste material fills the reservoir 14. As waste material fills the reservoir of the catheter 10, the body 12 of the catheter expands outwardly from a non-expanded condition to an expanded condition. Non-limiting examples of suitable elastomeric polymers include natural or synthetic rubbers, polyurethane, polyisoprene, polybutadiene, chloroprene, polyisobutylene, as well as combinations and copolymers thereof. By providing an expandable and resilient material to form the body 12, the body 12, due its natural ability to return to its non-expanded size/configuration, can assist in evacuation of waste material from within the reservoir 14 when the control valve 22 is opened.

In embodiments, the drain tube 20 supports an inner retainer member 30 and an outer retainer member 32 that are provided to fixedly secure the drain tube 20 to inner and outer surfaces of the abdominal wall 36 (FIG. 2). The retainer members 30 and 32 can be secured to the abdominal wall 36 using sutures (not shown) and can be formed from a variety of different biocompatible materials including absorbable or non-absorbable fasteners, tacks, glue, etc.

In embodiments, the catheter 10 includes a fluid sensor 40 that monitors the fill rate and/or level of waste material positioned within the reservoir 14 of the catheter 10 and notifies a patient when the reservoir 14 of the catheter 10 is filled and requires evacuation. The sensor 40 may be supported on an end of an indicator tube 42. Alternately, the sensor 40 can be positioned on the body 12 of the catheter 10 such that the sensor 40 directly communicates with the reservoir 14. When provided, the indicator tube 42 can be formed of a transparent material to facilitate visualization of waste material within the indicator tube 42. The fluid sensor 42 communicates with a control unit 44 which includes a processor (not shown) that processes or interprets the signal sent by the sensor 40 and a transmitter (not shown). The control unit 44 communicates with an indication device 46 positioned externally of the abdominal cavity of a patient. The indication device 46 may include a cell phone, a watch, a vibration device or any other type of visual display, tactile indicator or audible indicator.

Referring to FIG. 2, in use, the open end 16 of the body 12 of the catheter 10 is secured to an internal body vessel, e.g., the colon "C" such that waste material traveling through the colon "C" empties into the reservoir 14 of the catheter 10. The catheter 10 can be secured to the colon "C" using known fastening techniques including suturing, stapling or the like. The indicator tube 42 and the drain tube 20 are positioned to extend through the abdominal wall 36 (FIG. 2) and the body 12 of the catheter 10 is secured within a patient's abdomen "AB". As discussed above, the drain tube 20 is secured to inner and outer surfaces of the abdominal wall 36 with inner and outer retainers 30 and 32, respectively. The indicator tube 42 can be secured to the inner and outer surfaces of the abdominal wall 36 in a similar manner such that the sensor 40 is positioned adjacent an outer surface of the abdominal wall 36. Alternately, the sensor 40 can be secured within the abdominal cavity "AB". As discussed above, in some embodiments, the indicator tube 42 extends outwardly from the body 12 of the catheter 10 and is transparent such that waste material that flows into the indicator tube 42 can be visualized through the indicator tube 42.

When the reservoir 14 of the catheter 10 fills and reaches a level at which the waste material contacts the fluid sensor 40, the fluid sensor 40 transmits a signal to the control unit 44. The control unit 44 transmits a signal to an indication device 46 to alert the patient that the catheter 10 needs to be evacuated. In some embodiments, the indication device 46 may provide an audible, tactile or visual indication via an audible indicator or display positioned on or adjacent the sensor 40. In other embodiments, the control unit 44 wirelessly transmits a signal to the indication device 46, e.g., a watch or cell phone, carried by the patient to alert the patient that the catheter 10 requires evacuation.

When the patient is alerted that the catheter 10 requires evacuation, the control valve 22 is actuated to allow waste material to flow through the drain tube 20 from the reservoir 14. As discussed above, when waste material enters the reservoir 14, the resilient body 12 of the catheter 10 expands outwardly to apply an inwardly directed force on the waste material within the reservoir 14 that tends to urge the waste material from the reservoir 14 through the drain tube 20. Thus, when the control valve 22 is opened, movement of the body 12 from the expanded condition to the non-expanded condition forces the waste material from the reservoir 14.

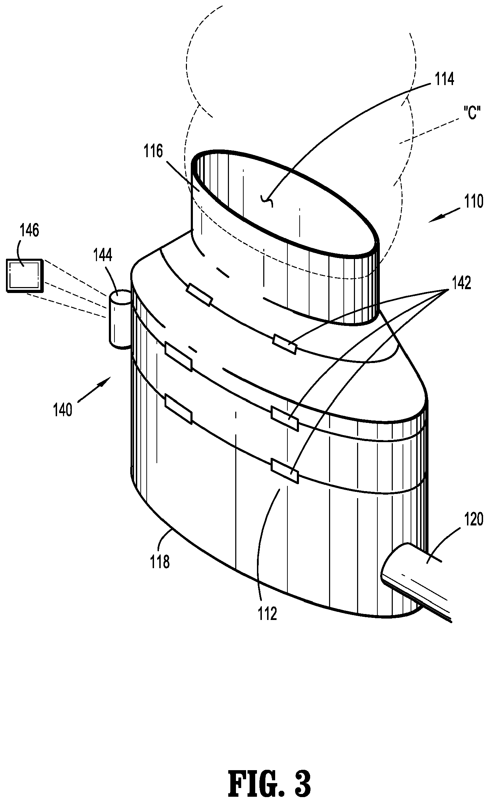

Referring to FIG. 3, in another exemplary embodiment of the presently disclosed internal colostomy catheter shown generally as catheter 110, the catheter 110 includes a body 112 which is similar to body 12 (FIG. 1) and is formed of an expandable and resilient material that defines a reservoir 114 and includes an open end 116 and a closed end 118. The body 112 includes a drain tube 120 having a control valve 22 (FIG. 2). In contrast to the catheter 10 shown in FIGS. 1 and 2, the sensor 140 includes strain gauges 142 and a control unit 144. The strain gauges 142 are positioned along the body 112. In embodiments, the strain gauges 142 can be positioned along inner or outer surfaces of the body 112 or can be embedded within the body 112.

In use, the catheter 110 is fixedly secured to the internal body vessel, e.g., the colon "C". As the waste material enters the reservoir 114, the strain gauges 142 measure expansion of the body 112 of the catheter 110 caused by waste material filling the reservoir 114. The strain gauge measurements are transmitted to the control unit 144 and the control unit 144 processes the measurements. When the strain gauge measurements exceed a predetermined value, the control unit 144 forwards the information to an indicator device 146, which may include a cell phone, a watch, a vibration device or any type of display or audible indicator, to alert the patient that evacuation of the catheter 110 is required.

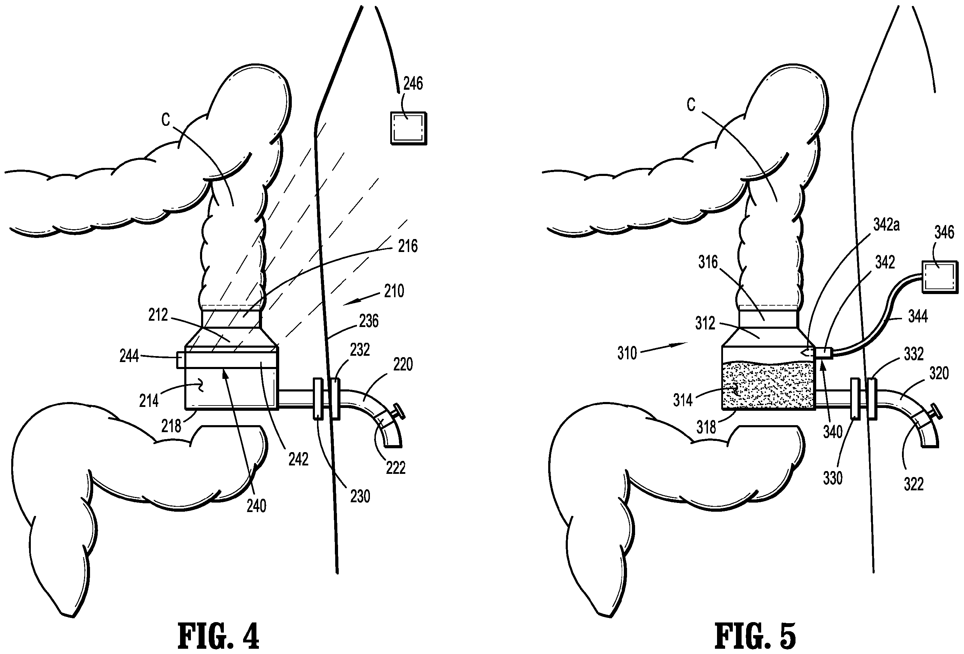

Referring to FIG. 4, in another exemplary embodiment of the presently disclosed internal colostomy catheter shown generally as catheter 210, the catheter 210 includes a body 212 similar to body 12 (FIG. 1), that is formed of an expandable and resilient material that defines a reservoir 214 and includes an open end 216 and a closed end 218. The body 212 includes a drain tube 220 having a control valve 222. In contrast to the catheter 10 shown in FIGS. 1 and 2, the sensor 240 includes a strain gauge 242 and a control unit 244. The strain gauge 242 is positioned about the body 212. In embodiments, the strain gauge 242 can be positioned along inner or outer surfaces of the body 212 or can be embedded within the body 212.

In use, the catheter 210 is fixedly secured to the internal body vessel, e.g., the colon "C". As waste material enters the reservoir 214 within the body 212, the strain gauge 242 measures expansion of the body 212 of the catheter 210 caused by the waste material filling the reservoir 214. The strain gauge measurements are transmitted to the control unit 244 and the control unit 244 processes the measurements. When the strain gauge measurements exceed a predetermined value, the control unit 244 forwards the information to an indicator device 246, which may include a cell phone, a watch, a vibration device or any type of visual display or audible indicator, to alert the patient that evacuation of the catheter 110 is required.

Referring to FIG. 5, in another exemplary embodiment of the presently disclosed internal colostomy catheter shown generally as catheter 310, the catheter 310 also includes a body 312 similar to body 12 (FIG. 1) formed of an expandable and resilient material that defines a reservoir 314 and an open end 316. The body 312 also includes a drain tube 320 having a control valve 322. In contrast to the catheter 10 shown in FIGS. 1 and 2, the sensor 340 includes an optical fluid level sensor 342 supported on the body 312 near the open end 316. The optical fluid level sensor 342 includes an infrared light emitting diode (LED) that is coupled with a light transistor, and a transparent prism tip 342a. The LED projects an infrared light outward. When the sensor tip 342a is surrounded by air, the infrared light from the LED reacts by bouncing back within the tip 342a before returning to the transistor. When the sensor tip 342a is immersed in waste material, the infrared light from the LED disperses within the waste material and less light is returned to the transistor. This change in degree of returned light is identified and transmitted through an output conduit 344 to activate an indicator device 346 which may include a cell phone, a watch, a vibration device or any type of display or audible indicator.

In use, the catheter 310 is fixedly secured to the internal body vessel, e.g., the colon "C". As waste material enters the reservoir 314 within the body 312, the optical fluid level sensor 342 is immersed in waste material filling the reservoir 314. When the sensor tip 342a of the optical fluid level sensor 342 becomes immersed in waste material and the infrared light from the LED disperses within the waste material, the output conduit 344 transmits this information to the indicator device 346 to alert the patient that evacuation of the reservoir 314 is required.

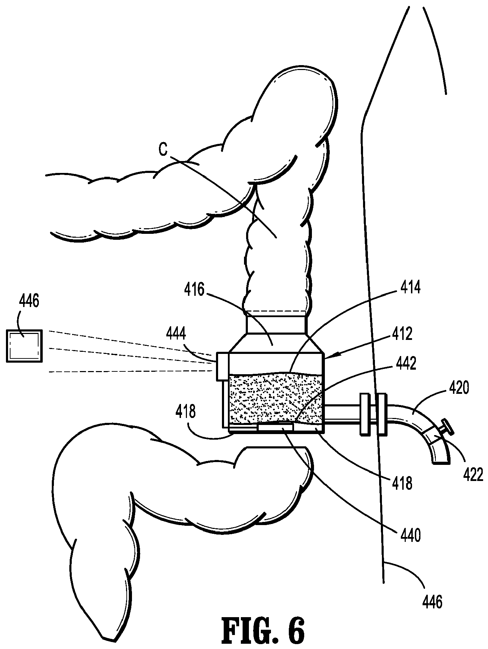

Referring to FIG. 6, in another exemplary embodiment of the presently disclosed internal colostomy catheter shown generally as catheter 410, the catheter 410 includes a body 412 similar to body 12 (FIG. 1) formed of an expandable and resilient material that defines a reservoir 414 and has an open end 416 and a closed end 418. The body 412 includes a drain tube 420 having a control valve 422. In contrast to the catheter 10 shown in FIGS. 1 and 2, the catheter 410 includes a pressure sensor or switch 440 supported on the body 412 near the closed end 418. A flexible separation wall 442 is positioned atop the pressure sensor 440 within the reservoir 414 such that the pressure sensor 440 is supported between the flexible separation wall 442 and the bottom wall. The pressure sensor 440 communicates with a control unit 444 and the control unit 444 communicates with an indicator device 446.

In use, the catheter 410 is fixedly secured to the internal body vessel, e.g., the colon "C". As waste material enters the reservoir 414 within the body 312, the weight of the waste material deflects the flexible separation wall 442. When the weight of the waste material reaches a predetermined level, the waste material activates the pressure sensor 440 which transmits a signal to the control unit 444. The control unit 444 processes the information received from the pressure sensor 440 and forwards the information to an indicator device 446 which may include a cell phone, a watch, a vibration device or any type of display or audible indicator to alert the patient that the catheter 410 needs to be evacuated.

In each of the embodiments of the presently disclosed internal colostomy catheter described above, the catheter body is secured to an internal body vessel to receive waste material from the internal body vessel. The catheter body supports a sensor that communicates with a control unit that processes signals from the sensor reflective of the volume of waste material received within the catheter. After processing the signals from the sensor that indicate that the catheter requires evacuation, the control unit forwards a signal to an indicator device to alert a patient that the evacuation of the catheter is required. This operation occurs while the catheter is supported within the abdominal cavity. The use of the presently disclosed embodiments of the internal colostomy catheter obviates the need for stoma formation and for an external colostomy bag.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described embodiments. Accordingly, the disclosure is not to be limited by what has been particularly shown and described, except as indicated by the appended claims.

The invention may be described by reference to the following numbered paragraphs:-
1. A surgical method comprising:
   securing a catheter to an internal body vessel within an abdominal cavity of a patient, the catheter having a body including an open end and a closed end and defining a reservoir, the open end being secured to the internal body vessel such that waste material from the internal body vessel is received within the reservoir;
   enclosing the catheter within the abdominal cavity;
   sensing the level of waste material within the reservoir with a sensor; and
   transmitting a signal from the sensor to an indication device positional externally of the abdominal cavity.
2. The surgical method of paragraph 1, further including evacuating waste material from the reservoir through a drain tube supported on the catheter when the level of waste material exceeds a predetermined amount.
3. The surgical method of paragraph 1, wherein the sensor includes a strain gauge positioned on the body of the catheter.
4. The surgical method of paragraph 3, wherein the body of the catheter is formed of an expandable material and the strain gauge measures expansion of the expandable material.
5. The surgical method of paragraph 1, wherein the sensor includes a fluid sensor supported on the body of the catheter.
6. The surgical method of paragraph 5, wherein the fluid sensor is supported on a transparent indicator tube that extends from the body of the catheter, and the method further includes analyzing the indicator tube to check for the presence of waste material.
7. The surgical method of paragraph 1, wherein the sensor includes an optical fluid level sensor supported on the body of the catheter adjacent the open end of the body of the catheter.
8. The surgical method of paragraph 1, wherein the sensor includes a pressure switch positioned on the closed end of the body of the catheter.
9. The surgical method of paragraph 1, wherein transmitting the signal from the sensor on the catheter to the indication device is done wirelessly.
10. An internal colostomy catheter comprising:
   a body defining a reservoir and having an open end and a closed end, the open end being configured and dimensioned to be secured to an internal body vessel;
   a hollow drain tube defining a discharge channel, the discharge channel communicating with the reservoir;
   a control valve positioned within the discharge channel;
   a sensor supported on the body to monitor a fluid level within the reservoir;
   a control unit for processing a signal received from the sensor and transmitting the signal; and
   an indication device adapted to receive the signal transmitted from the control unit and provide an indication related to the fluid level within the reservoir.
11. The internal colostomy catheter of paragraph 10, wherein the sensor a strain gauge positioned on the body of the catheter.
12. The internal colostomy catheter of paragraph 11, wherein body is formed of an expandable material and the strain gauge measures expansion of the expandable material.
13. The internal colostomy catheter of paragraph 10, wherein the sensor includes a fluid sensor supported on the body of the catheter.
14. The internal colostomy catheter of paragraph 13, wherein the fluid sensor is supported on a transparent indicator tube that extends from the body of the catheter.
15. The internal colostomy catheter of paragraph 10, wherein the sensor includes an optical fluid level sensor supported on the body of the catheter adjacent the open end.
16. The internal colostomy catheter of paragraph 10, wherein the sensor includes a pressure switch positioned on the closed end of the body of the catheter.
17. The internal colostomy catheter of paragraph 16, wherein the body includes a flexible separation wall positioned atop the pressure sensor within the reservoir.
18. The internal colostomy catheter of paragraph 10, wherein the control unit is adapted to transmit the signal to the indication device wirelessly.
19. The internal colostomy catheter of paragraph 10, wherein the indication device is selected from the group consisting of a watch and a cell phone.
20. The internal colostomy catheter of paragraph 10, wherein the indication device produces an audible or visual indication.

## Claims

1. An internal colostomy catheter comprising:
a body defining a reservoir and having an open end and a closed end, the open end being configured and dimensioned to be secured to an internal body vessel;
a hollow drain tube defining a discharge channel, the discharge channel communicating with the reservoir;
a control valve positioned within the discharge channel;
a sensor supported on the body to monitor a fluid level within the reservoir;
a control unit for processing a signal received from the sensor and transmitting the signal; and
an indication device adapted to receive the signal transmitted from the control unit and provide an indication related to the fluid level within the reservoir.

2. The internal colostomy catheter of claim 1, wherein the sensor includes a strain gauge positioned on the body of the catheter.

3. The internal colostomy catheter of claim 2, wherein the body is formed of an expandable material and the strain gauge measures expansion of the expandable material.

4. The internal colostomy catheter of any preceding claim, wherein the sensor includes a fluid sensor supported on the body of the catheter.

5. The internal colostomy catheter of claim 4, wherein the fluid sensor is supported on a transparent indicator tube that extends from the body of the catheter.

6. The internal colostomy catheter of any of claims 1 to 3, wherein the sensor includes an optical fluid level sensor supported on the body of the catheter adjacent the open end of the body of the catheter.

7. The internal colostomy catheter of any of claims 1 to 3, wherein the sensor includes a pressure sensor positioned on the closed end of the body of the catheter.

8. The internal colostomy catheter of claim 7, wherein the body includes a flexible separation wall positioned atop the pressure sensor within the reservoir.

9. The internal colostomy catheter of any preceding claim, wherein the control unit is adapted to transmit the signal to the indication device wirelessly.

10. The internal colostomy catheter of any preceding claim, wherein the indication device is selected from the group consisting of a watch and a cell phone.

11. The internal colostomy catheter of any preceding claim, wherein the indication device produces an audible or visual indication.
